# EUROPEAN PATENT APPLICATION

(11) **EP 2 374 899 A1**
(43) Date of publication of application: **12.10.2011**
(21) Application number: 10305354.2
(22) Date of filing: 07.04.2010
(51) Int. Cl.: C12Q 1/37, G01N 21/55, G01N 33/543, G01N 33/553

(54) **Efficient surface plasmon resonance based protease assay**

(71) Applicant: Idealp'Pharma, 69100 Villeurbanne (FR)
(72) Inventor: Yahiaoui, Farid, 69100 Villeurbane (FR); Petit, Serge, 69100 Villeurbanne (FR)
(74) Representative: Tetaz, Franck Claude Edouard

(57) **Abstract**

The present invention concerns an efficient surface plasmon resonance (SPR) protease assay which can be used for detect a protease activity in a media. The assay can be used for screen protease inhibitor or monitor biomarker activity. The high degree of efficiency of this assay is based on an innovative methodology, including the definition of an experimental strategy to improve assay efficiency by the use of a suitable couple antigen/antibody to improve the signal detection.

## Description

The present invention concerns an improved surface plasmon resonance (SPR) based enzymatic assay, wherein the mass-increasing moiety of the substrate used in the assay is an antigen coupled to an antibody. Use of a couple antigen/antibody provides an SPR assay with improved sensitivity.

### BACKGROUND OF THE INVENTION

Proteases play critical roles in many biological processes and are often good candidates for drug discovery. Detection of protease activity and screening of protease inhibitors have become crucial for the pharmaceutical industry, and a highly sensitive, fast and reliable protease assay method is needed for screening chemical compounds.

Today, a large variety of protease assay technologies have been developed. Most of them have its own advantages and disadvantages depending on the required screening parameters: sensitivity, throughput, cost (low consumption reagents)...

Typical assays for protease screening like fluorescence and chemiluminescence-based systems are founded on imperfect method that generates many false positive because the measures of the quantities of substrate and the end product of reaction are indirect and the molecular interferences are common. Indeed, these assays monitor the proteolysis by fluorescents signals, which can be disturbed by some components. Therefore it is necessary to explore the possibility to define a new methodology for develop new screening assay for protease inhibitors that would solve these limitations, with the possibility to detect directly the product of the enzymatic reaction.

WO 96/35806 discloses the general principle of a surface plasmon resonance (SPR) based enzymatic assay. The described method allows to measure directly the substrate and the end product of reaction by the monitoring of loss weight by SPR. The substrate and the protease are placed together in solution in a reaction vessel under conditions wherein the protease can cleave the substrate after which the reaction is stopped. The solution containing the protease and treated substrate are then brought into contact with a ligand bound to a sensor chip, wherein the ligand is able to bind to the substrate, and wherein the mass of the intact substrate versus the mass of the cleaved substrate is detected by SPR. The test substance is placed in solution in the reaction vessel with the protease and the substrate. The solution containing the protease, substrate and test substance are then brought into contact with a ligand bound to a sensor chip, wherein the ligand is able to bind to the substrate, and wherein the mass of the substrate versus the mass of the cleaved substrate is detected by SPR. If the substrate is cleaved as determined by its decrease in mass as detected by surface plasmon resonance technology then the test substance is not a protease inhibitor. On the other hand if the substrate is a protease inhibitor, then the substrate does not have a decrease in mass as is determined by surface plasmon resonance.

However the use of a couple biotin/streptavidin for increasing the signal detection severely limits the possibilities of using this assay in various media favourable for enzymatic reaction or favourable for SPR analysis (like running system buffer...) and severely limits the possibilities for use it as effective assay in terms of sensitivity, throughput and work concentration which must be the lowest. Indeed, increased SPR signal provided by the strategy of bio-affinity biotin/streptavidin is not sufficient to allow this assay to position itself as a relevant assay in terms of sensitivity and versatility compared to usual assays like fluorescence-based systems.

Indeed, it is necessary to have maximum signal detection according to the media injected in the SPR system. There is a need of an improved SPR method for establishing effective protease assay by SPR technology.

Inventors have defined a new methodology for this SPR assay, based on a strategic choice for the customized peptide-substrate and the mass-increasing moiety.

### SUMMARY OF THE INVENTION

The present invention fills this need by providing for an improved method to determine, with high sensitivity in different media, if a substrate is cleaved by an enzyme using SPR

The SPR method of the invention comprises the following steps of:
**(a)** providing a proteolytic enzyme substrate comprising a peptide that is modified at the amino-terminal or carboxy-terminal residue by synthetic attachment of a binding moiety and at the remaining amino-terminal or carboxy-terminal residue by synthetic attachment of a mass-increasing moiety, which peptide comprises a cleavage site recognized by the enzyme,
**(b)** treating the modified substrate of step (a) with a proteolytic enzyme under conditions in which proteolysis can occur,
**(c)** providing a sensor chip comprising a ligand coupled to it, which ligand is capable of reversibly binding to the binding moiety of the enzyme substrate of step (a),
**(d)** contacting the treated substrate of step (b) with the ligand of step (c) under the conditions in which the ligand can reversibly bind to the binding moiety and form a complex, and
**(e)** measuring the mass of the complex by surface plasmon technology, whereby measurement of a substantially lower mass for the treated substrate, compared to that of an untreated similar complexed substrate, indicates cleavage by the enzyme.

The SPR method of the invention is improved by using a couple antigen/antibody for the mass-increasing moiety.

Preferably the ligand is an antibody and the binding moiety an antigen.

The present invention is further comprised of a method for determining with high sensitivity and in various media, if a test substance is a protease inhibitor, by treating the substrate in step b) in presence of the test substance. The test substance can be added in sequence or simultaneously with the proteolytic enzyme.

The invention also concerns a kit for an improved SPR-based enzymatic/protease assay. The kit comprises all or part of the elements necessary to run the method of the invention with an SPR apparatus.

### DETAILLED DESCRIPTION OF THE INVENTION

Enzymatic assays with SPR technology are generally disclosed in WO 96/35806 and US 5,981,167 which contents are incorporated herein by reference.

The invention consists in establishing an efficient surface plasmon resonance protease assay from an innovative methodology, including the definition of an experimental strategy to improve assay efficiency and through the use of a suitable couple antigen/antibody for the mass-increasing moiety.

The mass-increasing moiety provides additional weight to the substrate when it is not cleaved. The mass-increasing moiety is attached to the substrate end opposite to the binding moiety. When the binding moiety is attached to the N-terminal end of the substrate, the mass-increasing moiety will be attached to the C-terminal end of the substrate and vice and versa. When the substrate is cleaved by the enzyme, a light moiety comprising part of the substrate and the binding moiety will bind to the ligand coupled to the sensor chip. On the other hand, when the substrate is not cleaved by the enzyme, a peptide comprising the mass-increasing moiety will bind to the ligand coupled to the sensor chip. Use of a mass-increasing moiety shall increase the weight difference between the uncleaved substrate and the cleaved substrate, therefore increasing the sensitivity of the method.

Such mass-increasing moieties are chosen not to interfere with the enzymatic reaction and comprise the antigen attached to the selected amino or carboxy-terminal end of the peptide and the corresponding antibody.

According to the invention, the antigen is either an antigen with a known antibody or one or more epitopes of the same antigen.

The antibody of the corresponding antigen is known in a sense the antibody is characterized and identified, not random. The antibody can be a polyclonal antibody or a monoclonal antibody, preferably a monoclonal antibody, more preferably purified.

The person skilled in the art will now how to select an appropriate antigen and to attach the selected antigen to the selected amino or carboxy terminal end of the peptide by conventional bonds, such as covalent bonds, particularly by amide bonds at the amino or carboxy-terminus of the peptide, possibly through spacer molecules.

In a particular embodiment of the invention, the antigen of the mass-increasing moiety is biotin. The antibody is advantageously a purified polyclonal anti-biotin antibody. Such purified anti-biotin antibodies are known in the art, including the antibody (antibiotin conjugate or not, antibiotin igG, antibiotin-HRP..., from goat, sheep, mouse, rabbit...in alkaline phosphatase, peroxidase, azide, glycerol...).

In the method of the invention, the antibody may be added to the reaction medium combined with the remaining of the substrate, before, during or after the treatment of the substrate with the proteolitic enzyme (step b). Preferably, the antibody of the mass increasing moiety is added to the reaction mixture after the treatment (step b), but it is possible to add the antibody after the immobilisation of the treted substrate on the chip (step d).

Various sensor chips with immobilized ligands are commercially available, and many more can be custom-made, as explained in Handbook of Surface Plasmon Resonance *(R.B.M. Schasfoort and Anna J. Tudos, 2008).* The ligand may be a protein, a polypeptide, more particularly an antibody, a DNA molecule, a RNA molecule, an oligonucleotide, a membrane, carboxymethylated dextran, carboxymethylated dextran pre-immobilized with streptavidin, alkanethiol molecules, lipophilic groups covalently attached to carboxymethylated dextran, carboxymethylated dextran pre-immobilized with nitrilotriacetic acid, etc...

Binding moieties for the above ligands are known in the art and the skilled person knows how to attach the binding moiety to the selected amino or carboxy terminal end of the peptide by conventional bounds, such as covalent bounds.

Preferably the ligand is an antibody and the binding moiety an antigen. When the binding moiety is an antigen, it must be preferably distinct from the antigen of the mass-increasing moiety, meaning that it does not bind to the same antibody as the antibody of the mass-increasing moiety.

The couple antigen/antibody for the binding moiety and the corresponding ligand may be a comprised with antigens known to be used in molecular biology to tag proteins or peptides, such as flag sequences, with their corresponding known antibodies.

In a particular embodiment of the invention, the antigen/binding moiety is a polyhistidine peptide, more particularly a hexahistidine peptide.

The antibody is a known antibody binding the polyhistidine peptide.

The person skilled in the art also knows how to coupling the antibody ligand to a sensor chip by known techniques used in protein purification and diagnostic methods based on antibody/antigen binding.

In a specific embodiment of the invention, we have worked with a commercial sensor chip with carboxymethylated dextran on surface. We have customised the chip with the irreversible immobilisation of an antibody to capture the treated substrate.

The enzyme substrate indeed depends on the proteolytic enzyme to be studied. The peptide comprising the cleavage site recognized by the enzyme in the substrate of the invention may be a protein or a peptide, part of the protein, known or identified to be cleaved by the enzyme to be studied. Such peptide shall generally have from 5 to 50 aminoacids, possibly more when a complete protein is used. A complete protein may be used when the actual cleavage site of the protein is not known or when the cleavage occurs only on the full length protein in the reaction medium.

The person skilled in the art will know the best substrate to be used in the method of the invention and the reaction conditions based on information available or obtained on the couple enzyme/substrate. The person skilled in the art can indeed adapt substrate and/or experimental protocol for an optimized sensibility.

According to a specific embodiment of the invention, the proteolytic enzyme is an enzyme identified to be involved in biological pathways where peptides and/or protein being cleaved by the said enzyme leads to a particular pathological condition for an animal or a plant. Therefore, identifying inhibitors of the proteolytic enzyme with the method of the invention would help finding products with a potential to prevent and/or cure the said pathological conditions.

Such proteolytic enzymes, their substrates and conditions in which proteolysis can occur are known in the art such as disclosed in the The Handbook of Proteolytic Enzymes (2003 Barrett A.J., Rawlings ND, Woessner JF.).

This method of the invention may be used for proteases involved in major diseases: the HIV-1 protease for AIDS; DPPIV for type 2 diabetes; thrombin, ACE proteases and renin for vascular disorders; cathepsin proteases for inflammation disorders; MMP proteases, caspases, etc.

The choice of the protease to be studied and the corresponding peptide comprising the cleavage site recognized by the enzyme is not a limiting factor to the present invention.

In a particular embodiment of the invention, the proteolytic enzyme is the aspartyl protease BACE-1. It is now recognized that a key role is played by the aspartyl protease BACE-1 that cleaves amyloid precursor protein (APP) to produce the amyloid ß42 peptide fragment, ultimately resulting in the formation of amyloid plaques. Development of specific inhibitors of BACE-1 proteases is thus one of the most active areas in the development of drugs for AD (Alzheimer disease).

The enzyme substrate is the amyloid precursor protein or a peptide fragment of the APP comprising the cleavage site of the APP by BACE-1. As disclosed above, the peptide fragment of the APP comprises form 5 to 50 aminoacids. More particularly, the said fragment comprises the following peptide: SEVNLDAEFR.

In a more specific embodiment, the peptide fragment of the APP comprises the following peptide: KTEEISEVNLDAEFRHD.

The substrate may also comprise spacer moieties between the peptide and the binding moiety and/or the antigen of the mass-increasing moiety. Spacers are chemical moieties, including amino acids, peptides, which role is to put away the peptide comprising the cleavage site recognized by the proteolytic and the binding moiety and/or the antigen of the mass-increasing moiety. Indeed the spacer is chosen not to interfere with the enzymatic reaction of cleavage of the peptide. The spacer may advantageously be an amino acid or a peptide, particularly a peptide which sequence is not comprised with the sequence of the enzyme substrate, more particularly comprising up to 5 amino acids.

According to a specific embodiment of the invention, the substrate consists in a following molecule:
Flag-(Spacer)ₙ-Peptide-(Spacer)ₙ₋Mass-increasing Moiety,
wherein Flag, Spacer, Peptide and Mass-increasing Moiety are defined above and in the examples and n is independently 0 or 1.

In more specific embodiment of the invention, the substrate consists in the following molecule:
(6His)-KTEEISEVNLDAEFRHD- {Lys(biotinyl)}
coupled with an anti-biotin antibody as defined above and in the example.

The method of the invention can be performed on any material or apparatus appropriate for surface plasmon resonance (SPR). Such apparatus as well known and available, such as Biacore systems (T100, A100, X100, 3000...), Bio-Rad system (Proteon XPR)...

The invention also provides for a method for determining if a test substance is a protease inhibitor. The method for identifying potential protease inhibitors of the invention comprises providing one or more substances to be tested, and performing the SPR method of the invention where treatment of the substrate in step b) is performed in presence of the test substance.

The test substance can be added in sequence or simultaneously with the proteolytic enzyme. The relative amount of test substance and the proteolytic enzyme can be modified to identify the potential inhibiting properties of the said substances at various concentrations.

Test substances comprise any type of substances susceptible to be used as protease inhibitor, including proteins or so called small molecules. The substances to be tested may be comprised in libraries, such as molecules libraries.

The proteolytic enzyme substrate comprising a peptide that is modified at the amino-terminal or carboxy-terminal residue by synthetic attachment of a binding moiety and at the remaining amino-terminal or carboxy-terminal residue by synthetic attachment of a mass-increasing moiety, which peptide comprises a cleavage site recognized by a proteolytic enzyme, wherein the mass-increasing moiety is a couple antigen/antibody as defined above and in the examples is also part of the present invention.

The invention also concerns a kit for an efficient SPR-based enzymatic/protease assay. The kit comprises all or part of the elements necessary to run the method of the invention with an SPR apparatus and particularly at least two, particularly three, of:
**(a)** a proteolytic enzyme substrate as defined above and in the example, and/or
**(b)** a proteolytic enzyme capable of cleaving the modified enzyme substrate, and/or
**(c)** a sensor chip comprising a ligand coupled to it, which ligand is capable of reversibly binding to the binding moiety of the modified enzyme substrate, and/or
**(d)** an antibody capable to bond to the mass increasing-moiety of the treated substrate.

All the elements of the kit are defined above and below in the examples. The kit shall comprise at least substrate and enzyme, or substrate and sensor chip, or substrate, enzyme and sensor chip.

In the kit of the invention, the antibody of the mass-increasing moiety may be presented apart from the rest of the ligand comprising the peptide, the binding moiety and the antigen of the mass-increasing moiety, as defined above and in the examples.

### FIGURES

Figure 1 : Optimum pH for a maximum immobilization of antibody.
Figure 2 : Stages for the covalent immobilization of the anti-polyHis Mab on the sensor chip.
Figure 3 : Peptide and streptavidin at different concentration.
Figure 4 : Sensorgram of a sample with BACE-1 peptide at 100nM and anti-biotin at 66nM.
Figure 5 : Repeatability of the measurements.
Figure 6 : Relative response depending on operating conditions (with or without activities).
Figure 7 : Signal evolution depending on the enzymatic parameters.
Figure 8 : Signal evolution depending on the enzymatic parameters.

### EXAMPLES

### Surface Plasmon Resonance Assay

The present example illustrates a method for establish an efficiency SPR protease assay by a high improvement of the signal detection. It allows to an effective assay for the detection and the monitoring of proteolytic activity of BACE-1.

All tests was performed on a *Biacore T- 100* SPR system from *GE Healthcare*

The BACE-1 peptide substrate was designed depending on the rate efficiency and the parameters of analysis like immobilisation and mass increasing. We have used a peptide with a His-Tag at the C-terminal and biotin at the N-terminal. The BACE-1 peptide is derived from the "Swedish mutation" of the amyloid precursor protein and is cleaved by BACE-1 between leucine and aspartic acid, as indicated (SEVNL/DAEFR)
*Formula of the BACE-1 peptide:*
(6His)-KTEEISEVNLDAEFRHD- {Lys(biotinyl)}

### Procedure for immobilize anti-polyhistidine Mab to the sensor chip SPR system parameters

*Sensor chip: CM5*

*Température: 25°C*

*Biacore running buffer: 1mM HEPES, 150mM NaCl, pH 7.4 with 3mM EDTA and 0.05% P20*

*Flow: variable*

The sensor chip, composed by a carboxymethylated dextran surface (CM5 product from *Biacore GE Healthcare),* is firstly treated at 5µL/min and 25°C by a solution of 1-ethyl-3-(3-dimethylaminopropyl) carbodiimide (EDC) and N-hydroxysuccinimide (NHS) to active the ester groups on the surface.

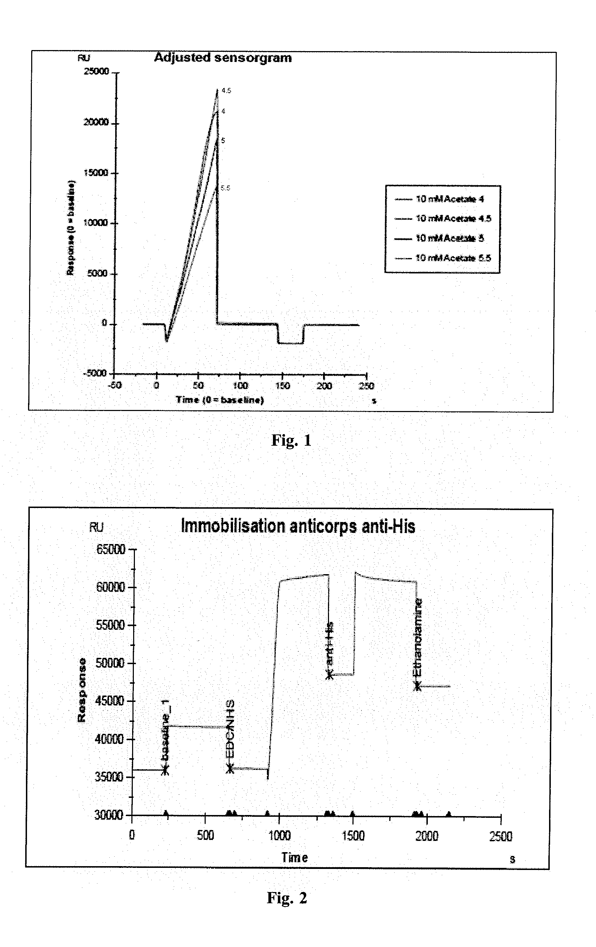

The anti-polyhistidine Mab at 50µg/ml is injected in 10mM acetate buffer pH=4.5 to covalent coupling with the active ester groups. It's lead to the immobilization of approximatively 11180 responses units of Mab. The acetate buffer pH=4.5 has been identified as optimal for maximum immobilization of the anti-polyhistidine Mab *(**fig.1**).*

For finish, ethanolamine is injected for block the non-react active ester.

Stages for this immobilization procedure are presented in figure 2 *(fig. 2**).*

### Mass increasing strategy for an enhancement of the SPR signal SPR system parameters

*Sensor chip: CM5*

*Temperature: 25°C*

*Biacore running buffer: 1mM HEPES, 150mM NaCl, pH 7.4 with 3mM EDTA and 0.05% P20*

*Flow: 40µl*/*min*

### Sample parameters

*Substrate: BACE-1 peptide*

*Temperature: 37°C*

*Buffer: Sodium acetate at pH=4.5*

*Mass increasing: anti-biotin PAb or streptavidin.*

To demonstrate the relevance of the use of a strategy *antigen on N-terminal*/*Tag antibody,* compared to other strategies like bio-affinity biotin/streptavidin, we retain a biotinylate group on N-terminal and we implement and compare the results obtained between streptavidin (as in the cited patent) and anti-biotin PAb.

We have prepared many samples with different concentration of BACE-1 peptide and streptavidin: 10µM of peptide is added to a sodium acetate buffer at ph=4.5 and for finish different among of streptavidin are added. The samples are diluted in the running buffer to obtain the desired concentrations and injected in the SPR system.

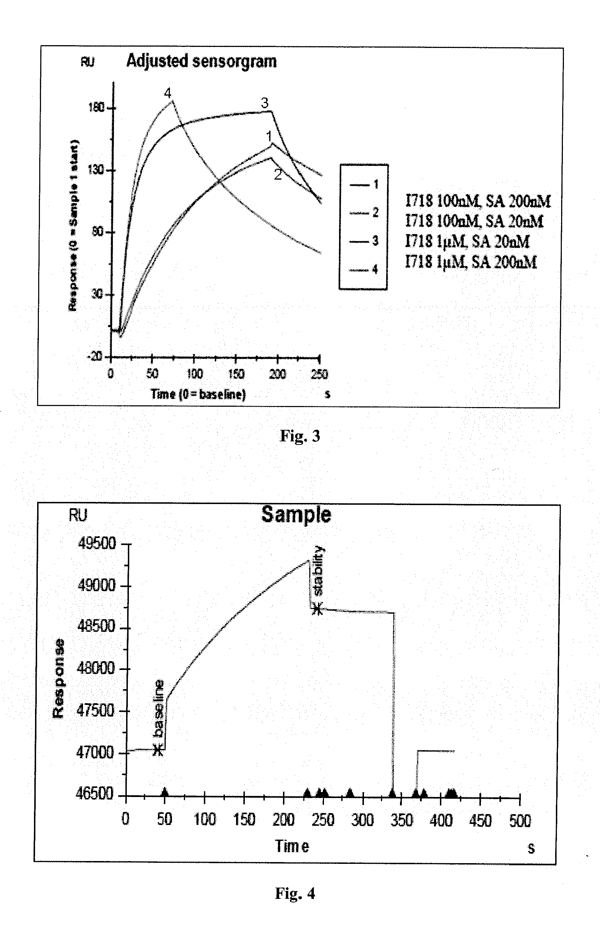

The results allowed us to estimate the optimal concentration of the peptide on the chip at approximatively 100nm and the optimal concentration of streptavidin (SA) is estimated at approximatively at 20nM *(**fig. 3**).* These two concentrations lead at a signal detection of approximatively 150 RU. In the same conditions, with the use of the same peptide concentration (100nm) in the same media, the use of 66nm of anti-biotin Pab, instead 100nm of SA, conducts to a signal detection of approximatively 1800 RU *(**fig.4**).* This antibody concentration is sufficient to quantify with high sensibility a proteolytic activity. We have defined this concentration as an optimal concentration (taking into account also the speed of analysis) but we have seen that at higher antibody concentrations, the SPR signal has been much higher.

The use of this strategy lead to a significant enhancement of the signal detection that can allow this assay to a sensitive and versatile assay necessary for detect an enzymatic activity in a complex media, like a biological media (cellular lysate...).

### Regeneration of the sensor chip

### SPR system parameters

*Sensor chip: CM5*

*Temperature: 25°C*

*Biacore running buffer: 1mM HEPES, 150mM NaCl, pH 7.4 with 3mM EDTA and 0.05% P20*

*Flow: 40µl*/*min*

### Sample parameters

*Substrate: BACE-1 peptide*

*Temperature: 37°C*

*Buffer: Sodium acetate at pH=4.5*

*Mass increasing: anti-polyHistine PAb*

For establish an effective assay screening, it is necessary to conduct an efficient regeneration for re-use the sensor chip many times without altering the properties of the immobilized antibody.

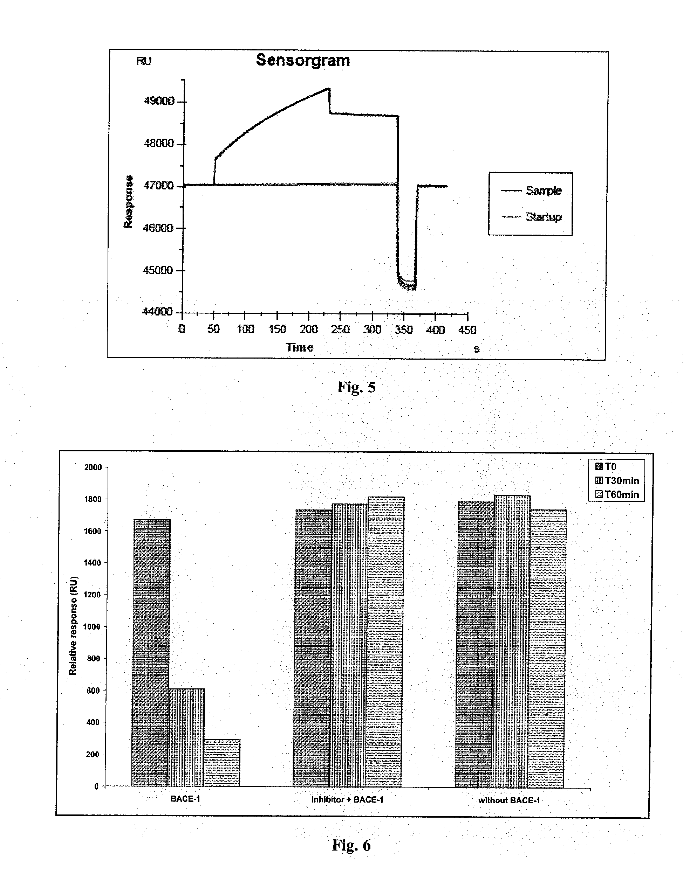

Usually, Glycine-HCl solution can effectively regenerate. However, we must find the optimal pH in the range 1.5-3.0 for a rapid removal of the peptide without alteration of the immobilized antibody on the sensor chip. From the results, we define the glycine-HC1 solution at pH=2.0 as the most appropriate solution for the desired regeneration: all the material captured on anti-polyHistidine Mab is released *(**fig.* 5).

To verify that the measured signal is only characteristic of the specific quantity of peptide bounded to the surface for each sample, for verify that the bounded peptide is correctly removed by the regeneration phase and for study the repeatability, we have injected consecutively, five times, the same solution containing antibiotin-tagged peptide. Between each injection, the surface was regenerated for 30 seconds with the same solution (Gly-HCl at pH=2.0).

The repeatability is excellent (CV∼0.2%) and the results showed that between each injection, the sensor chip does not deteriorate and that all the bounded peptide are removed by the regeneration. Then the measured SPR signal is specific to each sample.

### Application of described methodology to establish an effective assay for detect BACE-1 activity and screening its inhibitors

In recent years, the biological processes involved in the pathogenesis of Alzheimer's disease (AD) have become increasingly better understood. It is now recognized that a key role is played by the aspartyl protease BACE-1 that cleave amyloid precursor protein (APP) to produce the amyloid β42 peptide fragment, ultimately resulting in the formation of amyloid plaques. Development of specific inhibitors of BACE-1 proteases is thus one of the most active areas in the development of drugs for AD.

Thus, it is appropriate to define a new SPR-based screening assay for the identification of potential inhibitors for the aspartyl protease BACE-1 also applicable to other proteases.

We have used the described methodology for establish an effective method to detect BACE-1 activity in simplified media and screening its potential inhibitors. The method described allows us to quantify very finely the activity level of BACE-1, which allows therefore to screen with a high sensitivity enzyme inhibitors.

We have used the same BACE-1 peptide described before.

*Formula of the BACE-1 peptide:* (6His)-KTEEISEVNL-DAEFRHD-{Lys(biotinyl)}

The anti-polyHis Mab is immobilized on the sensor chip with same procedure described previously.

### Assay for detect BACE-1 activity and inhibition

### Typical Sample preparation

*Acetate buffer at pH=4.5*

*Addition of the BACE-1:final concentration 0.5U in 50µl of solution*

*Addition of the BACE-1 peptide: final concentration 10µM in 50µl of solution*

*Reaction at 37°C with various times (0, 30 or 60 minutes)*

*Quenching by addition of 1µl of specific BACE-1 inhibitors at 1µlM*

*Dilution of the sample in Biacore running buffer*

*Addition of the anti-biotin PAb*

*Dilution of the mixture in Biacore running buffer for have a sample injected into the SPR system with the BACE-1 peptide at 100nm and the anti-biotin at 66nm*

### SPR system parameters

*Sensor chip: CM5 with immobilized anti-polyhistidine Mab*

Temperature: 25°C

Biacore running buffer: 1mM HEPES, 150mM NaCl, pH 7.4 with 3mM EDTA and 0.05% P20

*Flow: 40µl*/*min*

*Regeneration with Gly-HCl solution at pH=2.0*

### Aim

To verify that the measured signal is characteristic of the BACE-1 activity and that this assay is able to detect the inhibitory effect of a compound, we have used an experimental plan with various operating conditions, described below.

### Experimental plan

We have defined three types of reaction. They are all conducted in parallel.

First group, with BACE-1. It is the typical reaction. The enzyme is present at 0.5U. The incubation is realized at 37°C and the reaction is stopped at 0, 30 or 60min (quench with the add of a specific inhibitor).

Second group, with a specific BACE-1 inhibitor. The inhibitor is added at t0 (five minutes before the introduction of the BACE-1 peptide) and at a concentration of 10µM.

Third group, without BACE-1. The same conditions are used but wit no enzyme. Thus, nine types of samples are prepared, as mentioned in *table 1* below.

**Table 1 : Samples analyzed**

| Incubation time (min) | With BACE-1 | Inhibited BACE-1 | Without BACE-1 |
|---|---|---|---|
| 0 | Sample 1 | Sample 4 | Sample 7 |
| 30 | Sample 2 | Sample 5 | Sample 8 |
| 60 | Sample 3 | Sample 6 | Sample 9 |

### Results

In the presence of the protease, the SPR signal decreases when the reaction time increases.

In the presence of a specific inhibitor or without BACE-1, the measured signals are approximately identical, like for the sample at t0: very high around 1800 RU, whatever the reaction times.

The results, shown in *fig* 6, clearly demonstrate that the developed assay is able to detect the activity of the BACE-1 protease, the measured responses are specific to the activity of the BACE-1 protease, and the developed assay is able to detect and quantify the inhibitory effect of a compound.

### Kinetic parameters

### Typical Sample preparation

*Acetate buffer at pH=4.5*

*Addition of the BACE-1: final concentration 0.25, 0.5U or 1U in 50µl of solution*

*Addition of the BACE-1 peptide: final concentration 10µM in 50µl of solution*

*Reaction at 37°C with various times (0, 15, 30 or 60 minutes)*

*Quenching by addition of 1µl of specific BACE-1 inhibitor at 1µlM*

*Dilution of the sample in Biacore running buffer*

*Addition of the anti-biotin PAb*

*Dilution of the mixture in Biacore running buffer for have a sample injected into the SPR system with the BACE-1 peptide at 100nm and the anti-biotin at 66nm*

### SPR system parameters

*Sensor chip: CM5 with immobilized anti-polyhistidine Mab*

*Temperature: 25°C*

*Biacore running buffer: 1mM HEPES, 150mM NaCl, pH 7.4 with 3mM EDTA and 0.05% P20*

*Flow: 40µ1*/*min*

*Regeneration with Gly-HCl solution at pH=2.0*

### Aim

To verify that the measured signal is characteristic of the BACE-1 activity and that this assay can detect significantly the level of activity of BACE-1, we used an experimental plan in which the parameters of the enzymatic reaction varies, as described below. The kinetic of the reaction was followed from the measurement of the SPR signal.

### Experimental plan

We have defined many types of reaction with various reaction time and protease concentration. They are all conducted in parallel.

For the first group, the reaction time is variable (0, 15, 30 or 60 minutes) and the enzyme concentration was set at 0.5U. The reaction is carried out at 37°C.

For the second group, the enzyme concentration is variable (0.25, 0.5 or 1.0U) and the reaction time was set at 60 minutes. The reaction is carried out at 37°C.

Thus, 12 types of samples are prepared, as mentioned in *table* 2 below.

**Table 2 : Samples analyzed**

| Incubation time (min) | BACE-1 concentration (Units) | | |
|---|---|---|---|
| | 0.25 | 0.5 | 1 |
| 0 | Sample 1 | Sample 5 | Sample 9 |
| 15 | Sample 2 | Sample 6 | Sample 10 |
| 30 | Sample 3 | Sample 7 | Sample 11 |
| 60 | Sample 4 | Sample 8 | Sample 12 |

### Results

We have observed that the signal is stable, whatever the concentration of the enzyme, since the reaction is stopped immediately (incubation time 0). This demonstrates that the BACE-1 peptide is stable over the time *(**fig* 6).

As expected, the signal decreases with highest incubation time or with highest enzyme concentration.

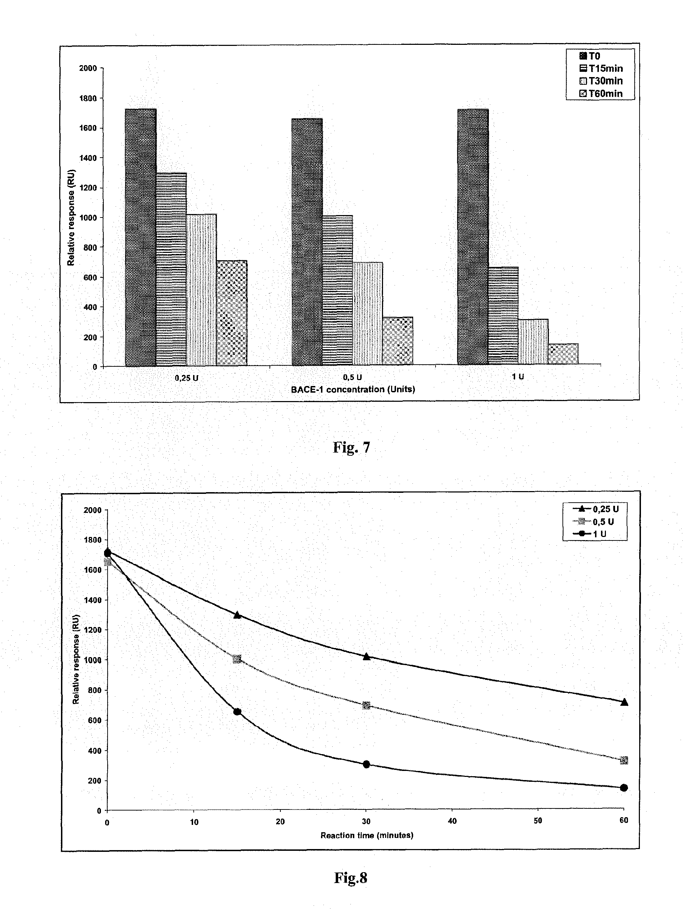

It was during the first 15 minutes that the enzyme is most active *(**fig* 7).

For each concentration, we have seen a decrease of the signal when the reaction time increases. It is characteristic of an enzymatic activity.

### Conclusion

We have presented the first described method for detect directly the BACE-1 activity and screen its inhibitory by a SPR-based protease assay.

We have established a method that allows us to screen inhibitors of BACE-1 by directly following the enzymatic activity of BACE-1 in simplified media.

The methodology allows us to screen with a high sensitivity from an amplification of the SPR signal and we can also work in a large variety of media.

The methodology developed also allows us to work with a throughput very important. Indeed, it is possible to analyze about 300 samples in one night, without having to change the sensor chip. This throughput may be substantially revised upwards by parallelizing measures, what can be done today with the new generation of SPR system.

We have demonstrated that by using a suitable methodology, it was possible to develop an efficiency assay for screen protease inhibitor by surface plasmon resonance.

### Materials

### Chemical Materials

*BACE-1 peptide was purchased from PolyPeptide Laboratories France SAS*

*BACE-1 protein was purchased from R&D Systems*

*The BACE-1 inhibitory was purchased from Calbiochem (BACE-1 inhibitor IV)*

*Protease buffer was composed of acetate buffer at pH=4.5*

*Biotin antibody (rabbit polyclonal) was purchased from Abcam*

*PolyHistidine antibody (mouse monoclonal) was purchased from R&D Systems*

*Streptavidin was purchased from Calbiochem*

### Surface plasmon resonance Materials

*SPR system was a Biacore T-100 from GE Healthcare*

*Sensor chip was a CM5 from GE Healthcare*

*All running buffer was purchased from GE Healthcare*

*Regeneration buffer was purchased from GE Healthcare.*

## Claims

1. A method to determine if a substrate is cleaved by a proteolytic enzyme using surface Plasmon resonance (SPR), comprising the steps of:
**(a)** providing a proteolytic enzyme substrate comprising a peptide that is modified at the amino-terminal or carboxy-terminal residue by synthetic attachment of a binding moiety and at the remaining amino-terminal or carboxy-terminal residue by synthetic attachment of a mass-increasing moiety, which peptide comprises a cleavage site recognized by the enzyme,
**(b)** treating the modified substrate of step (a) with a proteolytic enzyme under conditions in which proteolysis can occur,
**(c)** treating the modified substrate of step (b) with an antibody which bonds on the mass-increasing moiety of the treated substrate,
**(d)** providing a sensor chip comprising a ligand coupled to it, which ligand is capable of reversibly binding to the binding moiety of step (a),
**(e)** contacting the treated substrate of step (b) with the ligand of step (c) under the conditions in which the ligand can reversibly bind to the binding moiety and form a complex, and
**(f)** measuring the mass of the complex by surface plasmon resonance technology, whereby measurement of a substantially lower mass for the treated substrate, compared to that of an untreated similar complexed substrate, indicates cleavage by the enzyme, wherein the mass-increasing moiety is a couple antigen/antibody.

2. The method of claim 1, wherein the antigen of the mass-increasing moiety is biotin and the antibody a purified anti-biotin monoclonal antibody.

3. The method of one of claims 1 or 2, wherein the binding moiety is an antigen and the ligand is an antibody.

4. The method of claim 3, wherein the antigen binding moiety is distinct form the antigen of the mass-increasing moiety.

5. The method of one of claims 1 to 4, wherein the antibody of the mass-increasing moiety is added to the reaction medium combined with the remaining of the substrate, before, during or after the treatment with the proteolytic enzyme in step b).

6. The method of one of claims 1 to 5, wherein the proteolytic enzyme is the aspartyl protease BACE-1.

7. The method of claim 6, wherein the enzyme substrate is a fragment of the amyloid precursor protein (APP) comprising the peptide SEVNLDAEFR.

8. A method for identifying substances with potential protease inhibiting properties comprising providing one or more substances to be tested, and performing the SPR method of claims 1 to 7, wherein treatment of the substrate in step b) is performed in presence of the substance to be tested.

9. A proteolytic enzyme substrate comprising a peptide that is modified at the amino-terminal or carboxy-terminal residue by synthetic attachment of a binding moiety and at the remaining amino-terminal or carboxy-terminal residue by synthetic attachment of a mass-increasing moiety, which peptide comprises a cleavage site recognized by the enzyme, wherein the mass-increasing moiety is a couple antigen/antibody, the antigen being attached to the amino or carboxy-terminal residue of the peptide.

10. A kit for an improved SPR-based enzymatic/protease assay, said kit comprising at least two of:
**(a)** a proteolytic enzyme substrate comprising a peptide that is modified at the amino-terminal or carboxy-terminal residue by synthetic attachment of a binding moiety and at the remaining amino-terminal or carboxy-terminal residue by synthetic attachment of a mass-increasing moiety, which peptide comprises a cleavage site recognized by the enzyme, wherein the mass-increasing moiety is a couple antigen/antibody, the antigen being attached to the amino or carboxy-terminal residue of the peptide, and/or
**(b)** a proteolytic enzyme capable of cleaving the modified enzyme substrate, and/or
**(c)** a sensor chip comprising a ligand coupled to it, which ligand is capable of reversibly binding to the binding moiety of the modified enzyme substrate, and/or
**(d)** an antibody capable to bond to the mass increasing-moiety of the treated substrate.
